# EUROPEAN PATENT SPECIFICATION

(11) **EP 2 526 869 B1**
(45) Date of publication and mention of the grant of the patent: **21.12.2022**
(21) Application number: 12168381.7
(22) Date of filing: 16.05.2012
(51) Int. Cl.: A61B 6/02, A61B 6/12, A61B 90/11, A61B 90/17, A61B 10/02, A61B 17/34, A61B 6/00, A61B 17/00, A61B 34/10

(54) **IMAGE PROCESSING DEVICE, RADIOGRAPHIC IMAGE CAPTURE SYSTEM, IMAGE PROCESSING METHOD AND IMAGE PROCESSING PROGRAM STORAGE MEDIUM**
BILDVERARBEITUNGSVORRICHTUNG, RÖNTGENBILDERFASSUNGSSYSTEM, BILDERFASSUNGSSYSTEM, BILDVERARBEITUNGSVERFAHREN UND BILDVERARBEITUNGSPROGRAMMSPEICHERUNGSMEDIUM
APPAREIL DE TRAITEMENT D'IMAGES, SYSTÈME D'ACQUISITION D'IMAGES RADIOGRAPHIQUES, SYSTÈME D'ACQUISITION D'IMAGES, PROCÉDÉ DE TRAITEMENT D'IMAGES ET SUPPORT DE STOCKAGE DE PROGRAMME DE TRAITEMENT D'IMAGES

(30) Priority: 23.05.2011 JP 2011114851
(43) Date of publication of application: 28.11.2012
(73) Proprietor: FUJIFILM Corporation, Tokyo 106-8620 (JP)
(72) Inventor: TAJIMA, Takashi, Kanagawa (JP)
(74) Representative: Klunker IP Patentanwälte PartG mbB

(56) References cited:
- EP-A1- 1 844 725
- US-A1- 2005 033 160
- US-A1- 2007 019 781
- US-A1- 2009 274 271
- US-A1- 2009 326 553
- US-A1- 2010 063 496
- US-A1- 2010 121 316
- US-B2- 6 786 870

## Description

### BACKGROUND OF THE INVENTION

### Field of the Invention

The present invention relates to an image processing device, a radiographic image capture system, an image processing method and an image processing program storage medium, and in particular to an image processing device, a radiographic image capture system, an image processing method and an image processing program storage medium for inserting a biopsy needle into a subject to extract a tissue sample.

### Description of the Related Art

Biopsy examinations (biopsies) are performed in which a portion of an affected area of a patient is extracted for the purpose of medical diagnosis. In biopsies, targeting is performed by specifying locations from which a sample is desired (for example abnormal tissue) in radiographic images captured using a radiographic image capture device or in section (tomographic) images obtained by tomosynthesis imaging, and tissue is extracted.

In conventional targeting, one location is specified and a needle for biopsy (referred to below as a biopsy needle) is inserted into a patient such that the biopsy needle aperture reaches the location. Therefore, there have been cases in which a user, such as a doctor, cannot freely control the path of the biopsy needle, and there are difficulties in specifying the desired biopsy needle path, difficulties inserting the biopsy needle into the patient so as to avoid for example blood vessels, and difficulties in obtaining samples from plural nearby affected areas at the same time.

Technology to address this is known in which a user such as a doctor makes an instruction on a three-dimensional (3D) image captured by, for example, CT, MRI or tomosynthesis, the specified path is detected, and the biopsy needle insertion path is provided to the user by display on the 3D image.

For example, Japanese National-Phase Publication No. 2010-520006 describes a technology in which three-dimensional image data of a body is displayed, a target location is specified within the three dimensional image data by pointing and clicking over the image with a mouse, and a straight line (trial path) passing through the target within the 3D volume image data is designated.

Japanese Patent Application Laid-Open (JP-A) No. 2005-169070 describes a technology in which an insertion point where insertion is to be performed is specified while section images of the investigation subject are displayed, and based on this specification, an insertion path connecting the insertion point and the center of the affected region with a straight line is set.

JP-A No. 2007-209651 describes a technology in which an entry point of an instrument for insertion into a subject and the destination point inside the body of the subject are set on a three-dimensional image, obtained by stacking section (tomographic) images obtained by imaging the subject in a direction orthogonal to the plane of the sections, and determining an insertion path.

In biopsies, when a biopsy needle is inserted into a human body at an angle, precise extraction is not always possible when taking a sample of a target such as a lump or calcification in a breast. For example, in breast biopsies under stereo guidance, in which the targets of calcifications are minute compared to other locations, a target is specified on two radiographic images, and a biopsy needle driver device such as for example a biopsy unit inserts the biopsy needle into the human body (breast). The operation of a driver device such as a biopsy unit for driving a biopsy needle tends to depend on the structural limitations of the machine.

A user has to issue instructions on 3D images such as tomographic images whilst bearing such restrictions in mind.

### SUMMARY

The present invention provides an image processing device, a radiographic image capture system, an image processing method and an image processing program storage medium capable that enable more precise biopsy needle insertion into a human body.

The invention is defined by the independent claims. Preferred embodiments are defined by the dependent claims.

Plural radiographic images are captured for a site on a human body, which is a subject, by the radiographic image detection device irradiated with radiation from the radiation irradiation section that is provided facing the radiographic image detection device and irradiates radiation from plural positions as the radiation irradiation section is moved through the plural positions such that the radiation is irradiated at different angles with respect to the subject on the radiographic image detection device. The tomographic image generation means generates plural tomographic images of the subject, by reconstructing the plural radiographic images with reference to the detection face of the radiographic image detection device. The first specification means displays on the display means at least one tomographic image of the plural tomographic images in which an object of interest in the subject has been captured, so as to obtain specification from a user of the position of the object of interest in the at least one tomographic image based on the display. The second specification means displays on the display means at least one of the plural tomographic images including a region from the object of interest to a side from which a biopsy needle is to be inserted, according to the direction of insertion of the biopsy needle, so as to obtain specification from the user of a desired biopsy needle pass-through region and/or a non-pass-through region where it is undesirable for the biopsy needle to pass through based on the display.

After obtaining the user's specification of the location of the object of interest, and the desired biopsy needle pass-through region and/or the non-pass-through region, the detection means detects a path of the biopsy needle that passes through the pass-through region and reaches the object of interest, by setting the desired pass-through region as the pass-through region when the desired pass-through region has been specified, and setting a region other than the non-pass-through region as the pass-through region when the non-pass-through region has been specified. The warning means issues a warning when the path detected by the detection means is outside the predetermined movement range of the biopsy needle.

In biopsies, it is necessary to detect an insertion path for a biopsy needle that can precisely extract a sample of, for example, living tissue as the object of interest. In particular, in biopsies employing mammography in order to extract tissue from inside a breast, there are cases in which there is a heavy burden on a user for insertion path detection, since the insertion path is easily affected by limitations to the movement range due to the mechanical structure of the driving means for driving the biopsy needle.

In contrast, the present aspect can detect a biopsy needle insertion path by specifying on the tomographic image(s) the location of an object of interest and a region through which it is desired to pass the biopsy needle, and/or a region through which it is not desired to pass the biopsy needle, and in cases in which the detected path is outside of a predetermined movement range, it is possible to issue a warning. Therefore, more precise biopsy needle insertion can be made to the human body and the burden on a user is also reduced.

The present aspect may be configured such that the predetermined movement range includes a movement range predetermined according to capabilities of a driving means for driving the biopsy needle, a movement range predetermined such that the biopsy needle does not pass through a predetermined site in the imaging subject, or a combination thereof.

The present aspect may be configured such that the second specification means displays on the display means the tomographic image and a region representing the predetermined movement range.

The present aspect may be configured such that if plural objects of interest have been specified by the first specification means, the detection means detects a path that passes through the pass-through region and reaches at least two objects of interest among the plural objects of interest.

The present aspect may be configured such that if plural paths have been detected, the detection means sets an order of priority of the paths based on a predetermined criterion.

The present aspect may be configured such that if a region outside the predetermined movement range has been specified as the pass-through region by the second specification means, the detection means detects a path that does not pass through the pass-through region at the region outside the predetermined movement range.

A second aspect of the present invention is a radiographic image capture system including the features of claim 7.

A third aspect of the present invention is an image processing method including the features of claim 8.

A fourth aspect of the present invention is a non-transitory computer readable storage medium defined by claim 14.

According to the present aspects as described above, more precise biopsy needle insertion can be made to the human body.

### BRIEF DESCRIPTION OF THE DRAWINGS

Exemplary embodiments of the present invention will be described in detail based on the following figures, wherein:
Fig. 1 is a plan view illustrating an example of a configuration of a radiographic image capture device of the present exemplary embodiment;
Fig. 2 is a diagram illustrating an example of a configuration of the radiographic image capture device of the present exemplary embodiment during image capture;
Fig. 3 is an explanatory diagram to explain the radiographic image capture device of the present exemplary embodiment during image capture;
Fig. 4 is a block diagram illustrating an example of a configuration of a radiographic image capturing system of the present exemplary embodiment;
Fig. 5 is a flowchart illustrating an example of a flow of biopsy processing of the present exemplary embodiment;
Fig. 6 is a flowchart illustrating an example of a flow of insertion path detection processing executed by an image processing device of the present exemplary embodiment;
Fig. 7 is an explanatory diagram to explain a specific example of a pass-through region specifiable range in the insertion path detection processing of the present exemplary embodiment; and
Fig. 8 is an explanatory diagram to explain a specific example of displaying on tomographic images an insertion path detected by the insertion path detection processing of the present exemplary embodiment, illustrating a case in which the detection path is appropriate.

### DETAILED DESCRIPTION

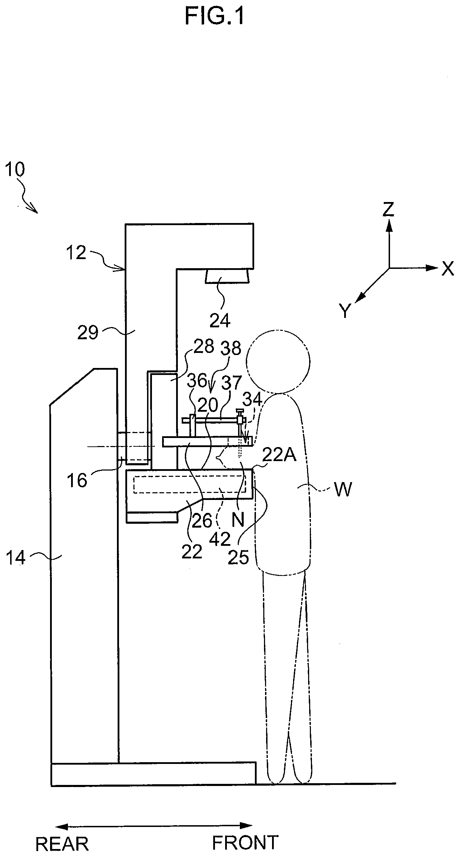

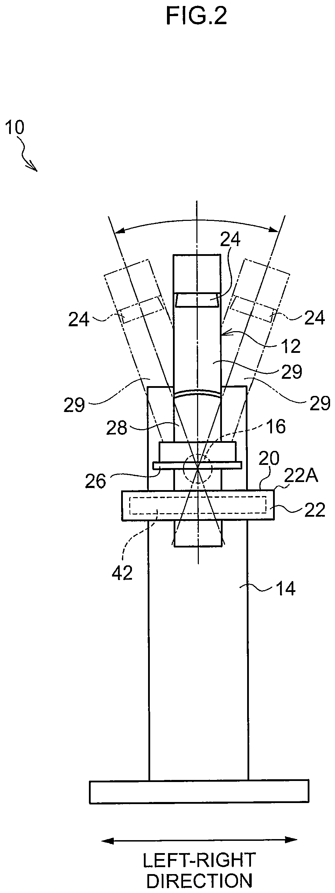

As shown in Fig. 1 to Fig. 3, a radiographic image capture device 10 of the present exemplary embodiment is a device for imaging a breast N of a subject W using radiation (for example X-rays), referred to as mammography, with the subject W in an upright posture. In the following explanation, the device front side of the radiographic image capture device 10 refers to the near side closest to the subject W when the subject W faces the radiographic image capture device 10 during imaging, the device rear side of the radiographic image capture device 10 refers to the far side away from the subject W when the subject W faces the radiographic image capture device 10 during imaging, and the device left and right directions of the radiographic image capture device 10 refer to the left and right directions for the subject W when the subject W faces the radiographic image capture device 10 during imaging (see the arrows in Fig. 1 and Fig. 2).

The imaging subject of the radiographic image capture device 10 is not limited to a breast N and, for example, other locations on the body or other objects may be the imaging subject. The radiographic image capture device 10 may also be configured as a device for imaging a breast N of a subject W with the subject W in a seated posture sitting on a chair (including a wheel chair). Configuration may be made with any device capable of imaging separately the left and right breasts N of a subject W with at least the upper body of the subject W in an upright posture.

The radiographic image capture device 10, as shown in Fig. 1, includes a measurement section 12 that is provided at the device front side and is substantially C-shaped when viewed from the side, and a base 14 that supports the measurement section 12 from the device rear side.

The measurement section 12 includes an imaging table 22 formed with a flat plane imaging face 20 that abuts the breast N of the subject W who is in an upright posture, a pressing plate 26 for pressing the breast N between the pressing plate 26 and the imaging face 20 of the imaging table 22, and a holder 28 for supporting the imaging table 22 and the pressing plate 26.

A biopsy hand section 38 is provided on the pressing plate 26 for taking a required tissue sample from the biopsy site of the breast N. A rectangular shaped opening 34 is provided in the pressing plate 26 for taking the tissue sample using the biopsy hand section 38. The biopsy hand section 38 includes a post section 36 fixed to the pressing plate 26 and an arm section 37 connected to one end of the post section 36. A biopsy needle 40 is mounted at the other end of the arm section 37. A sampler (not shown in the drawings) is provided at the tip portion of the biopsy needle 40 for sucking a tissue sample from the breast N biopsy site. The biopsy needle 40 can be moved by the biopsy hand section 38 in directions along the face of the pressing plate 26 (i.e., directions parallel to the pressing plate 26, that are the x-y axes direction in Fig. 1), and can also be moved in the direction of insertion into the breast N (i.e., directions intersecting with the pressing plate 26, that is the z axis direction in Fig. 1). The biopsy needle 40 in the present exemplary embodiment is configured capable of insertion into the breast N in a direction orthogonal to the pressing plate 26, and capable of insertion diagonally into the breast N. A member transparent to radiation is employed for the pressing plate 26.

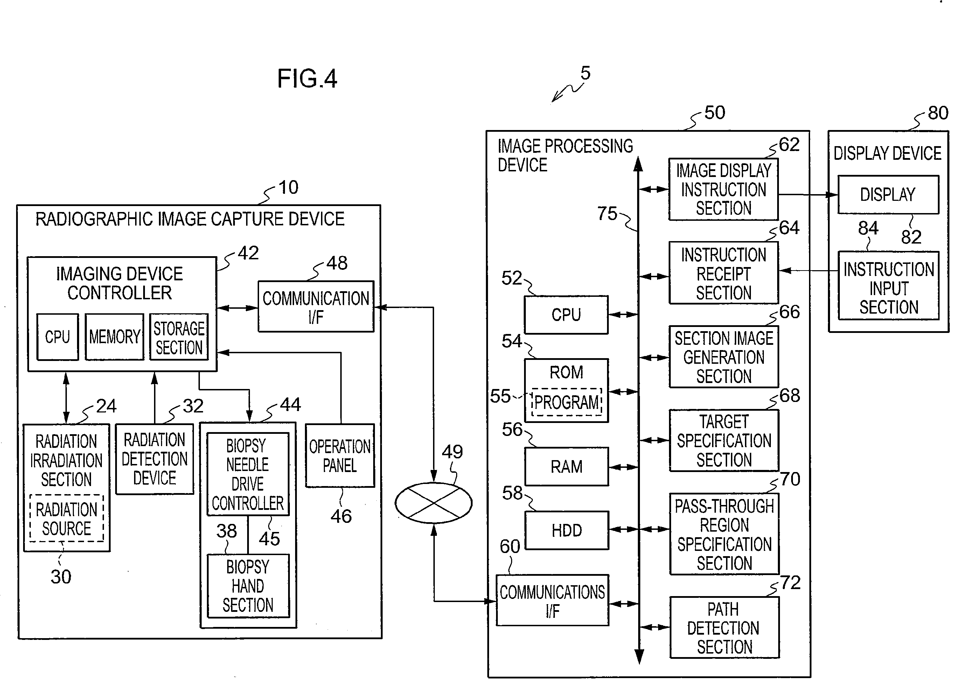

The measurement section 12 includes a radiation irradiation section 24 that is provided with a radiation source 30 such as a tube (see Fig. 4) for irradiating investigation radiation from the radiation source 30 towards the imaging face 20, and a support section 29 that supports the radiation irradiation section 24 independently of the holder 28.

The measurement section 12 is provided with a rotational movement shaft 16 rotatably supported by the base 14. The rotational movement shaft 16 is fixed to the support section 29 such that the rotational movement shaft 16 and the support section 29 rotate as a single unit.

The rotational movement shaft 16 is switchable between a state coupled and rotating as a unit with the holder 28, and a state rotating independently from the holder 28. More specifically, gears are provided to the rotational movement shaft 16 and the holder 28, and the gears can be switched between a meshed state and a non-meshed state.

Various mechanisms and elements may be employed to switch between transmission and non-transmission of rotation force of the rotational movement shaft 16 to the holder 28.

The holder 28 supports the imaging table 22 and the radiation irradiation section 24 such that the imaging face 20 and the radiation irradiation section 24 are at a specific separation from each other, and also retains the pressing plate 26 such that the pressing plate 26 can move by sliding to change the separation between the pressing plate 26 and the imaging face 20.

The imaging face 20 that contacts with the breast N is formed for example from carbon reinforced plastic from the perspectives of radiation permeability and strength. A radiation detection device 32 is disposed inside the imaging table 22. Radiation that has passed through the breast N and the imaging face 20 is irradiated onto and detected by the radiation detection device 32. The radiation detected by the radiation detection device 32 is made visible and a radiation image is generated.

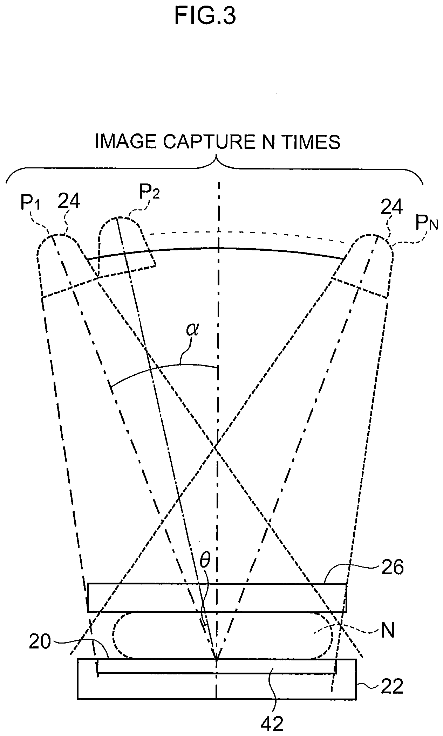

The radiographic image capture device 10 of the present exemplary embodiment is capable of at least performing imaging from plural directions for an imaging subject of a breast N. Fig. 2 and Fig. 3 illustrate orientations of the radiographic image capture device 10 during imaging and positions of the radiation irradiation section 24 during imaging. As shown in Fig. 2 and Fig. 3, such imaging performed by inclining the support section 29 that supports the radiation irradiation section 24 and that supports the imaging table 22 through the holder 28, during the imaging.

As shown in Fig. 3, when imaging of the breast N is performed from plural directions (tomosynthesis imaging) in the radiographic image capture device 10, the support section 29 is rotated while the rotational movement shaft 16 rotates free with respect to the holder 28 such that the imaging table 22 and the pressing plate 26 do not rotate. Consequently, only the radiation irradiation section 24 moves in a circular arc. In the present exemplary embodiment, as shown in Fig. 3, the imaging position is moved by steps of a specific angle θ at a time from angle α, and imaging is performed with the position of the radiation irradiation section 24 in N locations, P₁ to P_{N}.

The radiographic image capture device 10 of the present invention is capable of performing both Cranio and Caudal (CC) and Mediolateral-Oblique (MLO) imaging on a breast N. During CC imaging, the orientation of the holder 28 is adjusted to a state in which the imaging face 20 faces upward, and the orientation of the holder 28 is adjusted to a state in which the radiation irradiation section 24 is positioned above the imaging face 20. Radiation is thereby irradiated from the radiation irradiation section 24 onto the breast N from the head side to the feet side of the standing subject W so as to perform CC imaging. During MLO imaging, generally the orientation of the holder 28 is adjusted in a state in which the imaging table 22 is rotated by an angle of 45° up to 90° relative to during CC imaging, and a side wall corner portion 22A on the device front side of the imaging table 22 is positioned so as to abut the axilla of the subject W. Radiation is accordingly irradiated from the radiation irradiation section 24 to the breast N in a direction from the trunk axial center side of the subject W towards the outside, and MLO imaging is performed.

A chest wall face 25 is formed on the device front side face of the imaging table 22, and during imaging, the chest wall face 25 abuts the chest region below the breast N of the subject W. The chest wall face 25 is formed in a flat face.

Fig. 4 is a block diagram illustrating an example of a configuration of a radiographic image capture system 5 of the present exemplary embodiment.

The radiographic image capture system 5 of the present exemplary embodiment includes the radiographic image capture device 10, an image processing device 50 and a display device 80.

The radiographic image capture device 10 includes the radiation irradiation section 24, the radiation detection device 32, an imaging device controller 42, a biopsy unit 44, an operation panel 46, and a communication interface (I/F) 48.

The imaging device controller 42 has a function for controlling the overall operation of the radiographic image capture device 10, and includes a Central Processing Unit (CPU), memory including Read Only Memory (ROM) and Random Access Memory (RAM), a non-volatile memory section configured, for example, from a Hard Disk Drive (HDD) or a flash memory. The imaging device controller 42 is connected to the radiation irradiation section 24, the radiation detection device 32, the biopsy unit 44, the operation panel 46 and the communication I/F 48.

Upon receipt of an irradiation instruction from an operator using the operation panel 46 (radiation exposure switch), the imaging device controller 42 causes the radiation source 30 provided in the radiation irradiation section 24 to irradiate radiation on to the imaging face 20 following an imaging menu (described in detail later) which has been set based on specified radiation exposure conditions..

The radiation detection device 32 receives irradiated radiation that carries image data, stores the image data and outputs the recorded image data. The radiation detection device 32 is, for example, configured by a Flat Panel Detector (FPD) provided with a radiation sensitive layer that converts radiation into digital data. The radiation detection device 32 outputs image data expressing a radiographic image to the imaging device controller 42 when radiation has been irradiated. In the present exemplary embodiment, radiation that has passed through the breast N is received by the radiation detection device 32 and image data expressing a radiographic image is obtained.

The operation panel 46 functions to receive setting of various operation data, such as radiation exposure conditions and orientation data, and to receive various operation instructions.

The radiation exposure conditions set by the operation panel 46 include data regarding tube voltage, tube current, exposure duration and orientation data and the like. The orientation data specified with the operation panel 46 includes data indicating the imaging position (image capture orientation or angle) for capturing images of the breast N from plural directions.

These various operation data such as the radiation exposure conditions and orientation data and the various operation instructions may be set by an operator (user) using the operation panel 46, may be obtained from another control device (such as from a Radiology Information System (RIS) for managing data such as therapy and diagnosis data using radiology), and/or may be pre-stored in a storage section.

After various data has been set via the operation panel 46, the imaging device controller 42 executes radiographic image capture by causing radiation to be irradiated from the radiation irradiation section 24 onto the imaging site of the subject W (breast N) in accordance with an imaging menu, which has been set according to the various set data. When performing imaging from plural directions, the imaging device controller 42 adjusts the orientation of the holder 28 in a state in which the imaging face 20 faces upward, and adjusts the orientation of the support section 29 to a state in which the radiation irradiation section 24 is positioned above the imaging face 20. The imaging device controller 42 then, as shown in Fig. 3, rotates the support section 29, and moves the radiation irradiation section 24 in a circular arc in steps of angle θ from angle α. Radiation X are thereby irradiated according to the imaging conditions onto the imaging face 20 from the radiation source 30 provided in the radiation irradiation section 24 separately at each of the different angles. N frames of radiographic image are thereby obtained.

The biopsy unit 44 includes the biopsy hand section 38 and a biopsy needle drive controller 45. The biopsy needle drive controller 45 drives the biopsy unit 44 according to instructions from the imaging device controller 42, moves the biopsy needle 40 to a specific position, and holds the biopsy needle 40 in an inclined state at the insertion angle.

The communication I/F 48 has functions of sending and receiving captured radiographic images and various data to-and-fro between the radiographic image capture device 10 and the image processing device 50 via a network 49.

The image processing device 50 generates tomographic images by reconstructing the radiographic images acquired from the radiographic image capture device 10.

The image processing device 50 of the present exemplary embodiment detects the insertion path of the biopsy needle 40 based on the tomographic images, and instruct the radiographic image capture device 10 with the insertion path such that the biopsy needle 40 is inserted into the breast N along the detected insertion path.

The image processing device 50 is configured including a CPU 52, ROM 54, RAM 56, an HDD 58, a communication I/F 60, an image display instruction section 62, an instruction receipt section 64, a section (tomographic) image generation section 66, a target specification section 68, a pass-through region specification section 70 and a path detection section 72. These sections are linked together through a bus 75, such as a control bus or data bus, so as to enable data to be sent and received between each other.

The CPU 52 performs overall control of the image processing device 50, and more specifically perform control by executing a program 55 stored in the ROM 54. The program 55 in the present exemplary embodiment is pre-stored in the ROM 54; however, embodiments are not limited thereto and the program 55 may be stored on a storage medium such as a CD-ROM or removable disk and installed for example in the ROM 54 from the storage medium, or the program 55 may be installed for example in the ROM 54 from an external device via a communication line, such as the Internet. The RAM 56A secures a working region used when the CPU 52 executes the program 55. The HDD 58 stores and holds various data.

The communication I/F 60 has functions of transmitting and receiving captured radiographic images and various data between the image processing device 50 and the radiographic image capture device 10 via the network 49.

The image display instruction section 62 instructs a display 82 of the display device 80 to display images such as radiographic images (tomographic images).

The display device 80 of the present exemplary embodiment displays captured radiographic images (tomographic images), and is equipped with the display 82 for displaying radiographic images and an instruction input section 84. The instruction input section 84 allows a user (for example a doctor) who wishes to take a sample of an object of interest such as a calcification or lump to input instructions relating to display of radiographic images. Examples of the instruction input section 84 include a touch display, keyboard and mouse. In the present exemplary embodiment, the term "user" refers to someone such as a doctor who is taking a sample of an object of interest such as a lump or making a diagnosis using captured radiographic images, and the term "object of interest" refers to tissue that is targeted for a biopsy sample such as a calcification or lump.

The instruction receipt section 64 receives instructions input by a user with the instruction input section 84 of the display device 80.

The tomographic image generation section 66 reconstructs plural radiographic images obtained from tomosynthesis imaging and generates tomographic images parallel to the imaging face 20. In the present exemplary embodiment, the term "parallel" includes substantially parallel.

The tomographic image generation section 66 generates tomographic images from plural radiographic images I captured at positions P₁, P₂, P₃ to P_{N}. The position of an object of interest on a radiographic image differs according to the image capture angle for irradiating radiation from each position of the radiation source 30. The tomographic image generation section 66 therefore acquires from the radiographic image capture device 10 the image capture conditions during radiographic image capture, and computes a movement amount of the object of interest between the plural radiographic images based on the image capture angle included in the image capture conditions, and performs reconstruction of the plural radiographic images according to a known reconstruction method and generates tomographic images.

The target specification section 68 allows a user to specify at least one tissue site (target) for taking a biopsy sample in the breast N. In the present exemplary embodiment, tomographic images generated by the tomographic image generation section 66 are displayed on the display 82 of the display device 80 and a user may specify targets on the displayed tomographic images via the instruction input section 84 (further details are given later).

The pass-through region specification section 70 allows a user to specify a region in the breast N through which to pass the biopsy needle 40 when taking the sample at the target which has been specified by the target specification section 68. In the present exemplary embodiment, the tomographic images generated by the tomographic image generation section 66 are displayed on the display 82 of the display device 80 and the pass-through region can be specified by the user on the displayed tomographic images using the instruction input section 84 (further details are given later).

The path detection section 72 detects an insertion path of the biopsy needle 40 based on the position of the target specified by the target specification section 68 and the pass-through region specified by the pass-through region specification section 70.

Explanation follows regarding operation of the radiographic image capture system 5 of the present exemplary embodiment, with reference to the drawings.

Explanation first follows regarding the overall flow of operations when performing a biopsy using the radiographic image capture system 5, with reference to the drawings.

When performing a biopsy in the present exemplary embodiment, tomosynthesis imaging is first performed on the breast N of the subject W using the radiographic image capture device 10. Using the tomographic images obtained with the image processing device 50 by reconstructing the captured radiographic images, an insertion path for the biopsy needle 40 is detected based on specification from the user, and the insertion path is instructed to the radiographic image capture device 10. The radiographic image capture device 10 instructs the biopsy unit 44 to drive the biopsy hand section 38 under control of the biopsy needle drive controller 45 and to set the biopsy needle 40 according to the insertion path.

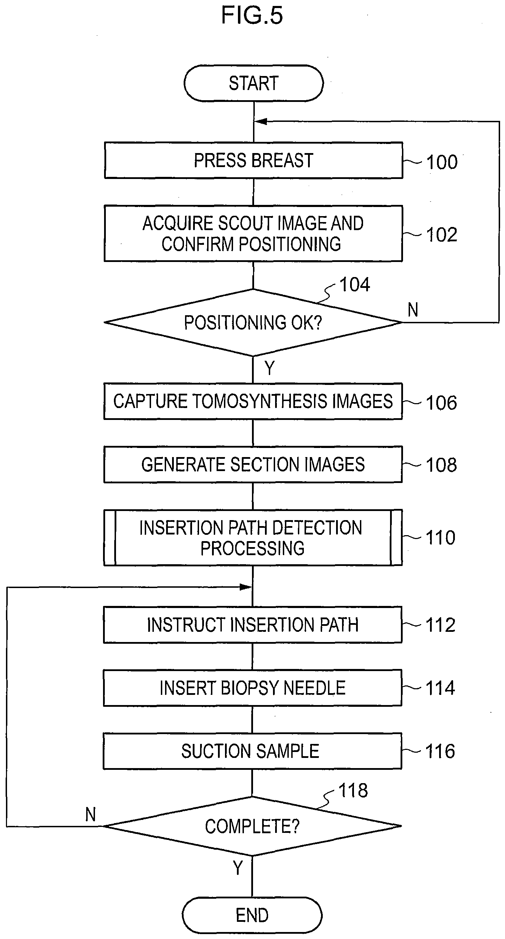

Fig. 5 shows a flow chart illustrating an example of processing flow during a biopsy.

When radiographic image capture is performed, an imaging menu is set in the radiographic image capture device 10 and imaging is executed according to the imaging menu.

In a case in which an imaging instruction has been input to the radiographic image capture device 10 to perform image capture of the breast N from plural directions, as shown in Fig. 2, the orientation of the holder 28 is adjusted to a state in which the imaging face 20 faces upwards and the orientation of the support section 29 is also adjusted to a state in which the radiation irradiation section 24 is positioned above the imaging face 20.

At step 100, the breast N of the subject W is pressed. The subject W places her breast N in contact with the imaging face 20 of the radiographic image capture device 10. In this state, the radiographic image capture device 10 moves the pressing plate 26 towards the imaging face 20 when a press start operation instruction is given by an operator on the operation panel 46.

Scout imaging is performed at the next step 102, a scout image is acquired, and the position of the subject W is confirmed. Scout imaging is imaging performed without rotating the radiation source 30, namely imaging with radiation irradiated at an angle of 0°. The operator determines whether or not the subject W is in an appropriate position with reference to the radiographic image captured by scout imaging.

At the next step 104, determination is made as to whether or not the position is appropriate. Processing returns to step 100 if the positioning is determined to be inappropriate, the pressing plate 26 is moved to release the pressing on the breast N, and the processing of step 100 to step 104 is repeated after repositioning the breast N. However, determination at step 104 is affirmative if the positioning is deemed appropriate, and the processing proceeds to step 106.

Tomosynthesis imaging is performed at step 106. In the radiographic image capture device 10 according to the present exemplary embodiment, in a case in which a tomosynthesis imaging instruction has been input to the operation panel 46 to perform imaging of the breast N from plural directions, only the support section 29 is rotated to move the radiation irradiation section 24 in a circular arc, and as shown in Fig. 3, the imaging position is moved from an angle α in steps of a specific angle θ. Radiation irradiation is performed based on image capture conditions with the position of the radiation irradiation section 24 at N individual locations, P₁ to P_{N}. The radiation thus separately irradiated from the radiation irradiation section 24 at plural positions arrives at the radiation detection device 32 after respectively passing through the breast N.

After radiation has been irradiated, the radiation detection device 32 outputs respective image data expressing irradiated radiographic images to the imaging device controller 42. In a case in which radiation has been irradiated with the position of the radiation irradiation section 24 at the N individual locations P₁ to P_{N}, image data for N frames of radiographic images is output to the imaging device controller 42.

The imaging device controller 42 outputs to the image processing device 50 the image data that has been input. In the case in which radiation irradiation has been performed as described above with the position of the radiation irradiation section 24 at the N individual locations P₁ to P_{N}, the CPU of the imaging device controller 42 outputs to the image processing device 50 image data for N frames of radiographic images.

Then, at step 108, tomographic images are generated by reconstructing the radiographic images by the image processing device 50. Tomographic images are generated in the image processing device 50 by reconstructing the N frames of radiographic images which have been input from the radiographic image capture device 10. The tomographic images are displayed on the display device 80.

At the next step 110, insertion path detection processing is performed by the image processing device 50. The image processing device 50 causes the display device 80 to displays the tomographic images thereon, allows a user to specify a tissue sampling target and a pass-through region through which the biopsy needle 40 is to pass. Insertion path detection processing (described in detail later) is then performed based on these specifications to detect the insertion path of the biopsy needle 40.

At step 112, the image processing device 50 instructs the radiographic image capture device 10 with the detected insertion path. After the insertion path has been instructed, the radiographic image capture device 10 instructs the biopsy unit 44 with the insertion path. After the insertion path has been instructed, the biopsy needle drive controller 45 drives the biopsy hand section 38 and moves the biopsy needle 40 to a position and angle corresponding to the insertion path.

Then, at step 114, the biopsy needle 40 is inserted into the breast N. In the present exemplary embodiment, insertion of the biopsy needle 40 into the breast N using the biopsy unit 44 is performed by a user such as a doctor. After the biopsy needle 40 has been inserted, at step 118, target tissue is suctioned (resected) and sampled by the inserted biopsy needle 40.

At step 118, determination is made as to whether or not processing is complete. The determination is negative if there is another target tissue for sampling in the breast N, and the processing returns to step 112 and repeats the processes of reinserting the biopsy needle 40 according to the specified insertion path and suctioning target tissue. If the determination is affirmative at step 118, the processing is ended.

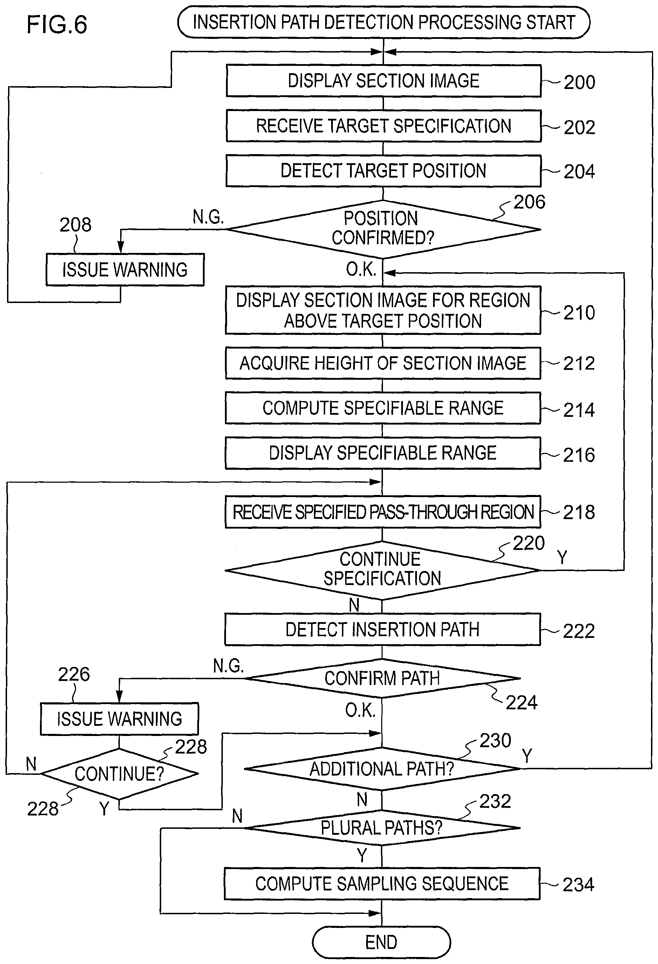

Detailed explanation follows regarding the insertion path detection processing of above step 110. Fig. 6 shows a flow chart of an example of the flow of insertion path detection processing executed by the image processing device 50 of the present exemplary embodiment. The processing is performed by executing the control program 55 stored in memory by the CPU 52.

At step 200, the generated tomographic images are displayed by the display 82 of the display device 80 for a user to specify a target with the target specification section 68. Configuration may be made such that a single frame or successive single frames of tomographic images are displayed on the display device 80, or such that plural tomographic images are displayed on the display device 80 at the same time.

A user may specify a target (object of interest) for tissue sampling on the tomographic images displayed on the display 82 of the display device 80 using the instruction input section 84.

At step 202, the specification of the target from the user is received by the instruction receipt section 64. Next, at step 204, the position of the target (3-dimensional coordinates) is detected based on the tomographic images, and determination is made as to whether or not the position of the target is appropriate at step 206. The determination here is whether or not the position of the target is within a control range of the biopsy unit 44 of the radiographic image capture device 10. In the present exemplary embodiment, data related to the control range of the biopsy unit 44 is pre-stored in a storage section, not shown in the drawings, of the image processing device 50. If the position of the target is not within the control range of the biopsy unit 44, the determination is negative and the processing proceeds to step 208. At step 208, the user is warned that the position of the target is outside the control range of the biopsy unit 44. In the present exemplary embodiment, for example, warning is performed by display on the display device 80. However, the method of warning is not limited thereto, and the user may be warned using sound or light, through any devices such as the display device 80.

If the position of the target is within the control range of the biopsy unit 44, the determination is affirmative and the processing proceeds to step 210. At step 210, at least one tomographic image which shows regions above the position of the target is displayed by the pass-through region specification section 70 on the display 82 of the display device 80 for a user to specify the insertion path of the biopsy needle 40.

Then, at step 212, the height position of the tomographic image being displayed on the display device 80 (the position of the tomographic image in the z axis direction) is acquired, and at step 214, a specifiable range is computed within which specification by a user is enabled. In the present exemplary embodiment, the specifiable range is where the extension line of the insertion path does not cross (go beyond) the chest wall of the subject W and is also within the control range of the biopsy unit 44. Whether or not the extension line of the insertion path crosses the chest wall of the subject W is computed with reference to the insertion path. In the present exemplary embodiment, the region beyond the chest wall (the body region inside the chest wall) is not regarded as the region of the breast N (i.e., is regarded as body tissue other than breast N). Namely, the chest wall is taken as the boundary of the breast N. Other organs such as the heart and lungs are present in the region inside the chest wall. Therefore, in the present exemplary embodiment, the region beyond the chest wall is regarded as being outside of the specifiable range in order to prevent the biopsy needle from reaching regions other than the breast N. The determination as to whether or not the position of the target is inside the control range of the biopsy unit 44 is computed from the height positions of the tomographic images, the 3-dimensional coordinates of the target and the angle of the biopsy needle 40.

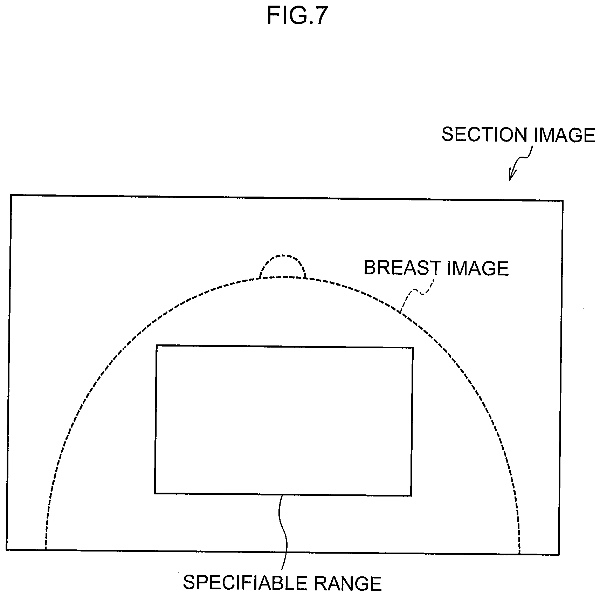

At step 216, the computed specifiable range is displayed on the tomographic image being displayed on the display device 80. A specific example of a display of the specifiable range is shown in Fig. 7.

The user may specify a desired pass-through region of the biopsy needle 40 in the tomographic image displayed on the display 82 of the display device 80 using the instruction input section 84. Generally, a user does not prefer the biopsy needle 40 to pass through a region where there are blood vessels or other lesions where biopsy is not to be performed. The user therefore specifies the desired pass-through region of the biopsy needle 40 in consideration of the regions where pass-through is undesirable. Configuration may be made such that the desired pass-through region is specified as the pass-through region, or the non-pass-through region is specified where pass-through is undesirable and regard the regions outside the non-pass-through regions as the pass-through region.

In the next step 218, the specified pass-through region is received by the instruction receipt section 64.

At step 220, determination is made as to whether or not to continue pass-through region specification. The determination is affirmative if the user wishes to specify a pass-through region using another tomographic image at a different height (position in the z axis direction), and the processing returns to step 210 and the processing for specifying a pass-through region is repeated. However, the determination is negative if the user does not wish to continue specification, and the processing proceeds to step 222.

At step 222, the insertion path is detected by the path detection section 72 based on the specified position of the target and the specified pass-through region. In the present exemplary embodiment, the insertion path is computed using a least squares method from the 3-dimensional coordinates of the target and the 3-dimensional coordinates of the pass-through region. However, the computation method is not particularly limited and may be any computation based on the specified position of the target and the specified pass-through region.

In a case in which a user has specified a range outside the specifiable range as the pass-through region, detection of the insertion path may be performed by excluding the pass-through region that is outside of the specifiable range. Preferably, a warning is given to a user when an insertion path is detected that passes through a region outside of the pass-through region specified by the user.

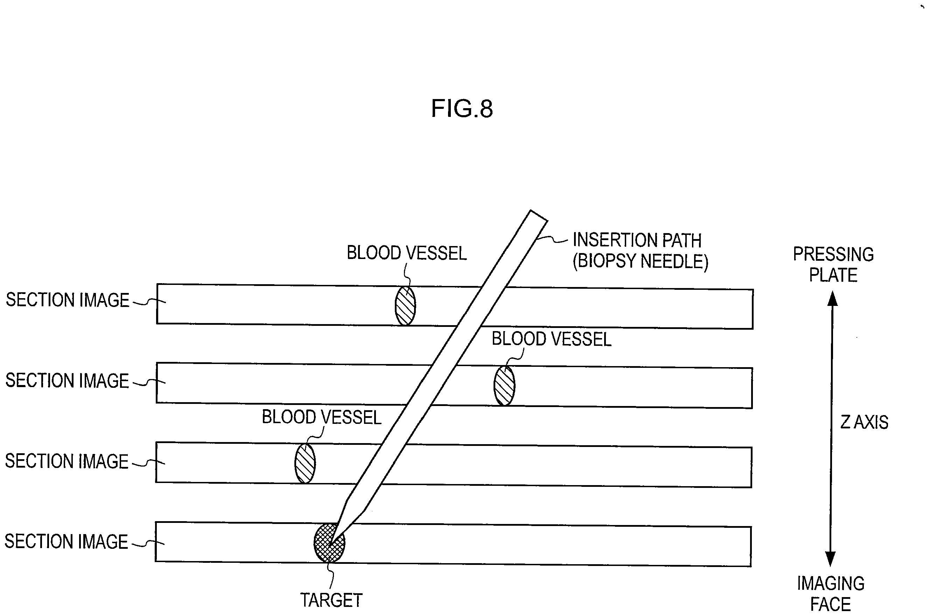

At the next step 224, confirmation is made as to whether or not the detected insertion path is appropriate. In the present exemplary embodiment, a tomographic image in which the insertion path is indicated is displayed on the display device 80 for a user's confirmation. Whether or not the insertion path is appropriate is also confirmed by the path detection section 72 of the image processing device 50. In the present exemplary embodiment, the determination is inappropriate if the extension line of the insertion path exceeds the chest wall of the subject W, if the 3-dimensional coordinates and angle of the biopsy needle 40 are outside of the control range of the biopsy unit 44, if the biopsy needle 40 passes through a blood vessel and/or the tip of the biopsy needle 40 reaches a blood vessel. Whether or not the biopsy needle 40 passes through a blood vessel may be determined by performing detection using wavelet transformation based on the tomographic images as described in for example JP-A No. 2004-313478; however, the method is not particularly limited thereto. A specific example of an insertion path determined appropriate is illustrated in Fig. 8. Processing proceeds to step S230 if the insertion path is determined appropriate (including a case of receiving a confirmation from a user).

However, the processing proceeds to step 226 if the insertion path is determined to be inappropriate (including a case of not receiving a confirmation from a user), and a user is warned that the path is inappropriate at step 226. In the present exemplary embodiment, for example, a warning is displayed on the display 82 of the display device 80. However, the method of warning is not limited thereto, and the user may be warned using sound or light, through any devices such as the display device 80. The method of warning may be changed according to a predetermined criterion.

The present exemplary embodiment is configured such that the reason the insertion path being determined to be inappropriate is displayed on the display device 80. For example, even if it is detected that the insertion path passes through a blood vessel, there are cases in which a user determines that taking the target sample is more important than the anticipated blood loss arising from the biopsy needle 40 passing through the blood vessel. In such cases, the user may decide that there is still a need to perform a biopsy even though the insertion path is determined to be inappropriate in the determination of step 224. Therefore, in the present exemplary embodiment, the user is prompted to decide whether or not to perform a biopsy using the insertion path (whether or not to continue the processing) when the warning is given.

A the next step 228, determination is made as to whether or not to continue the processing. Determination is negative if the instruction receipt section 64 receives an instruction not to continue the processing, and the processing then returns to step 218, where specification of the pass-through region and detection of the insertion path is repeated. However, the determination is affirmative if an instruction to continue the processing is received by the instruction receipt section 64, and the processing proceeds to step 230.

At step 230, determination is made as to whether or not to add an additional path. Determination as affirmative for example in cases in which the user desires to take a tissue sample by inserting the biopsy needle 40 from two or more different directions into a single target, and in cases in which the user desires to take a tissue sample from another target, and the processing returns to step 200 and the subsequent processes are repeated. However, the processing proceeds to step 232 if determination is negative.

At step 232, determination is made as to whether or not there are plural insertion paths. Determination is negative if there is only a single insertion path and the current processing is ended. However, determination is affirmative if there are plural insertion paths and the processing proceeds to step 234.

At step 234, a sampling sequence is computed, and the current processing is ended after determining the sequence of use of the insertion paths (the sequence for taking samples of the targets). In the present exemplary embodiment, an order of priority for insertion paths is pre-stored in a storage section, not shown in the drawings, and the sequence for target sampling is determined according to the order of priority. An example of the order of priority is, for example, assigning a maximum priority to not passing through a blood vessel, then sequencing from the shortest biopsy needle insertion path (the shorter the insertion path the higher the order of priority). The reason the order of priority is higher the shorter the biopsy needle insertion path in this example is in consideration of the expectation that the amount of blood loss is smaller with a shorter insertion path when the biopsy needle 40 has passed through a blood vessel. Determination of the sampling sequence is however not limited thereto, and configuration may be made such that the sampling sequence is determined based on a user' setting.

By thus determining the sampling sequence based on the order of priority, for example, since any insertion paths that pass through blood vessels are last in the sequence, sampling requiring operations such as mopping up lost blood or sampling in which stopping blood loss takes a long time can be performed at the end. Further, in cases in which sampling is performed for successive targets, spilt blood fluids can be prevented from obscuring targets for which sampling is performed later.

As described above, in the radiographic image capture system 5 of the present exemplary embodiment, when a biopsy is performed on a region of interest of the breast N of the subject W, radiation is irradiated from different angles onto the breast N of the subject W by the radiographic image capture device 10, thereby performing imaging of plural radiographic images, which is referred to as tomosynthesis imaging. Tomographic images are generated by reconstructing the plural radiographic images in the tomographic image generation section 66 of the imaging processing device 50. The target specification section 68 displays the generated tomographic images on the display 82 of the display device 80. After the instruction receipt section 64 receives the position of a target specified by the user based on the displayed tomographic images, the pass-through region specification section 70 displays, on the display 82 of the display device 80, at least one tomographic image showing regions above the position of the specified target in the z axis direction (regions at the side from which the biopsy needle 40 is to be inserted, i.e., the pressing plate 26 side in the present exemplary embodiment). The present exemplary embodiment computes the specifiable range as a range where the insertion path does not go beyond the chest wall and the insertion path is within the control range of the biopsy unit 44. The specifiable range is displayed together with the tomographic images on the display device 80. After the image processing device 50 receives an instruction of a desired pass-through region of the biopsy needle 40, which has been specified by the user based on the displayed at least one tomographic image, the path detection section 72 detects the insertion path of the biopsy needle 40 using the position (3-dimensional coordinates) of the target and the position (3-dimensional coordinates) of the pass-through region. The path detection section 72 displays the insertion path on the display 82 of the display device 80 for a user's confirmation of whether or not it is an appropriate insertion path, and determines whether or not the insertion path is appropriate according to predetermined conditions, such as whether or not the insertion path goes beyond to the chest wall, the insertion path is within the control range of the biopsy unit 44, and/or whether or not the insertion path passes through a blood vessel. The image processing device 50 warns the user if it is determined that the insertion path is inappropriate. Further, if plural insertion paths have been detected, a sampling sequence for the targets is determined based on the predetermined order of priority and the insertion paths are instructed to the radiographic image capture device 10. The radiographic image capture device 10 controls the biopsy unit 44 to perform biopsies based on the instructed insertion path(s) and the insertion sequence.

As described above, the image processing device 50 allows a user to specify the position of targets for tissue sampling in the tomographic images displayed on the display device 80, and then allows the user to specify a desired pass-through region of the biopsy needle 40 on the tomographic images representing regions at the side from which the biopsy needle 40 is to be inserted than the specified target, which are displayed on the display device 80. The image processing device 50 detects the insertion path of the biopsy needle 40 using the 3-dimensional coordinates of the specified target and the 3-dimensional coordinates of the pass-through region, determines as to whether or not the detected insertion path is appropriate, and warns the user if the insertion path is determined to be inappropriate. As a result, an insertion path for the biopsy needle 40 can be detected in consideration of the control range of the biopsy unit 44 and the breast N region. Accordingly, biopsy needle insertion can performed to the human body with increased accuracy.

Since a user is only required to specify the target and the pass-through region on the displayed tomographic images, the burden on a user to detect the insertion path is reduced.

Further, depending on the insertion angle and insertion path, the biopsy needle 40 may pass near the chest wall or is inserted towards the chest wall when employing mammography to perform a biopsy on a breast N. However, the present exemplary embodiment can draw attention of a user by determining the appropriateness of an insertion path and issuing a warning so that the biopsy needle 40 does not exceed the chest wall and reach a region other than the region of the breast N. Furthermore, by displaying the insertion path on the display device 80, the user can be made aware of the distance between the position of the biopsy needle 40 and the chest wall.

In the present exemplary embodiment, since the specifiable range where the pass-through region is allowed to be specified is displayed on the display device 80 together with the tomographic images as described above, a user can be prevented from unintentionally specifying a pass-through region outside the specifiable range and receiving a warning. Path specification is accordingly facilitated and user's operability is enhanced.

In the present exemplary embodiment, the burden on a user is thus reduced, and since an insertion path can be detected appropriately within a short period of time, the time for an investigation can be shortened, and the burden on the subject W can be reduced.

In the present exemplary embodiment, explanation has been given of a case in which the insertion path is detected by the path detection section 72. However, configuration may be made for example such that a user is informed by display on the display device 80 in a case in which an insertion path cannot be detected, and the user made to specify a new pass-through region.

Furthermore, in the present exemplary embodiment, tomographic images showing regions which are positioned above the target are displayed since the biopsy needle 40 is inserted from above to below with respect to the imaging face 20 (in a direction approaching towards the imaging face 20). However, the embodiment is not limited thereto in a case in which the insertion direction of the biopsy needle 40 is different. The configuration may be such that tomographic images are displayed that show regions further toward the side from which the biopsy needle 40 is to be inserted (the side external to the subject W) than the target (i.e., the region through which the biopsy needle 40 will possibly pass).

In the present exemplary embodiment, explanation has been given of a case in which there is a single target for a single insertion path; however, embodiments are not limited thereto and configuration may be made such that an insertion path is determined such that plural targets can be sampled at the same time. In such cases, if it is not possible to determine a single insertion path that can sample all the plural specified targets, configuration may be made such that a insertion path is determined capable of sampling most of the targets, and/or insertion paths are determined such that the number of times of biopsy needle 40 insertion required to take all the samples is as small as possible. These determinations may be displayed on the display device 80.

In the present exemplary embodiment, explanation has been given of a case in which tomographic images of radiographic images captured by mammography are employed to detect the insertion path; however, embodiments are not limited thereto and radiographic images captured by another radiographic image capture device may be employed. The location for performing a biopsy is also not limited to the breast N. However, as explained above, the present exemplary embodiment is preferably applicable to use in biopsy from the breast N since precise sampling of minute targets such as lumps or calcifications in the breast N is important.

The radiation employed to capture radiographic images is also not particularly limited, and for example X-rays or γ-rays may be employed.

The configurations described in the present exemplary embodiment of the radiographic image capture system 5, the radiographic image capture device 10, the image processing device 50 and the display device 80 are examples, and obviously various changes are possible according to the circumstances within a scope not departing from the spirit of the present invention.

The flow of biopsy processing and flow of insertion path detection processing described in the present exemplary embodiment are also examples thereof, and obviously various changes are possible within the scope of the invention as defined by the appended claims.

## Claims

1. An image processing device comprising:
a tomographic image generation means configured to generate a plurality of tomographic images of an imaging subject, which is a site on a human body, by reconstructing a plurality of radiographic images acquired from a radiographic image detection device using a detection face of the radiographic image detection device as a reference, the plurality of radiographic images being acquired by moving a radiation irradiation section facing the radiographic image detection device through a plurality of positions and irradiating the imaging subject disposed on the radiographic image detection device with radiation from each of the plurality of positions such that the radiation is irradiated at different angles with respect to the imaging subject;
a first specification means configured to display on a display means at least one tomographic image of the plurality of tomographic images, in which an object of interest in the imaging subject has been captured, and that allows a user to specify a position of the object of interest in the at least one tomographic image based on the display;
a second specification means configured to display on the display means at least one of the plurality of tomographic images including a region from the object of interest to a side from which a biopsy needle is to be inserted based on the position of the object of interest and a direction of insertion of the biopsy needle, and allows the user to specify, based on the display, a desired pass-through region of the biopsy needle, a non-pass-through region through which it is undesirable for the biopsy needle to pass, or a combination thereof;
a detection means configured to detect a path of the biopsy needle passing through the pass-through region and reaching the object of interest, wherein the desired pass-through region is regarded as the pass-through region if a desired pass-through region has been specified, and a region other than the non-pass-through region is regarded as the pass-through region if a non-pass-through region has been specified; and
a warning means configured to issue a warning if the detected path is outside a predetermined movement range of the biopsy needle.

2. The image processing device of claim 1, wherein the predetermined movement range includes a movement range predetermined according to capabilities of a driving means for driving the biopsy needle, a movement range predetermined such that the biopsy needle does not pass through a predetermined site in the imaging subject, or a combination thereof.

3. The image processing device of claim 1 or claim 2, wherein the second specification means is configured to display, on the display means, the tomographic image and a region representing the predetermined movement range.

4. The image processing device of any one of claim 1 to claim 3, wherein, if a plurality of objects of interest have been specified by the first specification means, the detection means is configured to detect a path that passes through the pass-through region and reaches at least two objects of interest among the plurality of objects of interest.

5. The image processing device of any one of claim 1 to claim 4, wherein, if a plurality of paths has been detected, the detection means is configured to set an order of priority of the paths based on a predetermined criterion.

6. The image processing device of any one of claim I to claim 5, wherein, if a region outside the predetermined movement range has been specified as the pass-through region by the second specification means, the detection means is configured to detect a path that does not pass through the pass-through region at the region outside the predetermined movement range.

7. A radiographic image capture system comprising:
a radiographic image capture device comprising:
a radiographic image detection device,
a radiation irradiation section configured to face the radiographic image detection device, and configured to irradiate radiation from each of a plurality of positions while moving through the plurality of positions such that the radiation is irradiated at different angles with respect to an imaging subject disposed on the radiographic image detection device, and
a driving means configured to drive a biopsy needle for taking a sample of an object of interest in the imaging subject; and
the image processing device of any one of claim 1 to claim 6, configured to delete a path of the biopsy needle based on to the plurality of radiographic images captured by the radiographic image capture device.

8. An image processing method comprising:
generating a plurality of tomographic images of an imaging subject, which is a site on a human body, by reconstructing a plurality of radiographic images acquired from a radiographic image detection device using a detection face of the radiographic image detection device as a reference, the plurality of radiographic images being acquired by moving a radiation irradiation section facing the radiographic image detection device through a plurality of positions and irradiating the imaging subject disposed on the radiographic image detection device with radiation from each of the plurality of positions such that the radiation is irradiated at different angles with respect to the imaging subject;
displaying on a display means at least one tomographic image of the plurality of generated tomographic images in which an object of interest in the imaging subject has been captured, and receiving a first specification of a position of the object of interest in the at least one tomographic image, which is specified based on the display;
displaying on the display means at least one of the tomographic images including a region from the object of interest to a side from which a biopsy needle is to be inserted based on the specified position of the object of interest and a direction of insertion of a biopsy needle, and receiving a second specification of a desired pass-through region of the biopsy needle, a non-pass-through region through which it is undesirable for the biopsy needle to pass, or a combination thereof, which is specified based on the display;
detecting a path of the biopsy needle passing through the pass-through region and reaching the object of interest, wherein the desired pass-through region is regarded as the pass-through region if a desired pass-through region has been specified, and a region other than the non-pass-through region is regarded as the pass-through region if a non-pass-through region has been specified; and
issuing a warning when the detected path is outside a predetermined movement range of the biopsy needle.

9. The image processing method of claim 8, wherein the predetermined movement range includes a movement range predetermined according to capabilities of a driving means for driving the biopsy needle, a movement range predetermined such that the biopsy needle does not pass through a predetermined site in the imaging subject, or a combination thereof.

10. The image processing method of claim 8 or claim 9, wherein the displaying of the at least one tomographic image comprises displaying the tomographic image and a region representing the predetermined movement range on the display means.

11. The image processing method of any one of claim 8 to claim 10, wherein, if a plurality of objects of interest have been specified in the first specification, the detecting comprises detecting a path that passes through the pass-through region and reaches at least two objects of interest among the plurality of objects of interest.

12. The image processing method of any one of claim 8 to claim 11, wherein, if a plurality of paths have been detected, the detecting comprises setting an order of priority of the paths based on a predetermined criterion.

13. The image processing method of any one of claim 8 to claim 12, wherein if a region outside the predetermined movement range has been specified as the pass-through region in the second specification, the detecting comprises detecting a path that does not pass through the pass-through region at the region outside the predetermined movement range.

14. A non-transitory computer readable storage medium that stores a program which, when executed by a device according to claim 1, causes said device to perform the method according to claim 8.

## Patentansprüche

1. Bildverarbeitungsvorrichtung, umfassend:
eine Tomographiebild-Erzeugungseinrichtung, konfiguriert zum Erzeugen einer Mehrzahl von Tomographiebildern eines Bildobjekts, das eine Stelle in einem menschlichen Körper ist, durch Rekonstruieren einer Mehrzahl von Radiographiebildern, die von einer Radiographiebild-Detektoreinrichtung unter Verwendung einer Detektorfläche der Radiographiebild-Detektoreinrichtugn als Referenz erfasst wurden, wobei die mehreren Radiographiebilder erfasst wurden durch Bewegen eines der Radiographiebild-Detektoreinrichtung gegenüberliegenden Strahlungsabgabeabschnitts über eine Mehrzahl von Positionen und Bestrahlen des auf der Radiographiebild-Detektoreinrichtung angeordneten Bildobjekts mit Strahlung aus jeder der Mehrzahl von Positionen derart, dass die Strahlung unter verschiedenen Winkeln bezüglich des Bildobjekts aufgestrahlt wird;
eine erste Spezifikationseinrichtung, konfiguriert zum Anzeigen mindestens eines Tomographiebilds der mehreren Tomographiebilder auf einer Anzeigeeinrichtung, wobei in dem angezeigten Tomographiebild ein interessierendes Objekt in dem Bildobjekt aufgenommen wurde, und das es einem Benutzer ermöglicht, eine Position des interessierenden Objekts in dem mindestens einen Tomographiebild anhand der Anzeige zu spezifizieren;
eine zweite Spezifikationseinrichtung, konfiguriert zum Anzeigen mindestens eines der mehreren Tomographiebilder einschließlich einer Zone aus dem interessierenden Objekt zu einer Seite, aus der eine Biopsienadel eingeführt wird, auf der Anzeigeeinrichtung, basierend auf der Position des interessierenden Objekts und einer Einführrichtung der Biopsienadel, und die es dem Benutzer ermöglicht, basierend auf der Anzeige eine Soll-Durchstechzone der Biopsienadel, eine durchstichfreie Zone, durch die die Biopsienadel nicht hindurchstechen sollte, oder eine Kombination davon zu spezifizieren;
eine Detektoreinrichtung, konfiguriert zum Detektieren eines Wegs der durch die Durchstechzone hindurchgehenden Biopsienadel mit Erreichen des interessierenden Objekts, wobei die Soll-Durchstechzone als eine Durchstechzone betrachtet wird, wenn eine gewünschte Durchstechzone spezifiziert wurde, und eine von der durchstichfreien Zone verschiedene Zone als eine Durchstechzone betrachtet wird, wenn eine durchstichfreie Zone spezifiziert wurde;
und eine Warneinrichtung, konfiguriert zum Abgeben einer Warnung dann, wenn der ermittelte Weg außerhalb eines vorbestimmten Bewegungsbereichs der Biopsienadel liegt.

2. Bildverarbeitungsvorrichtung nach Anspruch 1, bei der der vorbestimmte Bewegungsbereich einen Bewegungsbereich enthält, der nach Maßgabe der Möglichkeiten einer Treibereinrichtung zum Antreiben der Biopsienadel festgelegt wurde, einen Bewegungsbereich, der so vorbestimmt wurde, dass die Biopsienadel nicht durch einen vorbestimmten Ort in dem Bildobjekt hindurchgeht, oder eine Kombination derselben.

3. Bildverarbeitungsvorrichtung nach Anspruch 1 oder Anspruch 2, bei der die zweite Spezifikationseinrichtung konfiguriert ist, um auf einer Anzeigeeinrichtung das Tomographiebild und eine Zone anzuzeigen, welche den vorbestimmten Bewegungsbereich repräsentiert.

4. Bildverarbeitungsvorrichtung nach einem der Ansprüche 1 bis 3, bei der dann, wenn eine Mehrzahl von interessierenden Objekten durch die erste Spezifikationseinrichtung spezifiziert wurde, die Detektoreinrichtung konfiguriert ist zum Detektieren eines Wegs, der durch die Durchstechzone hindurchgeht und mindestens zwei interessierende Objekte unter den mehreren interessierenden Objekten erreicht.

5. Bildverarbeitungsvorrichtung nach einem der Ansprüche 1 bis 4, bei der dann, wenn eine Mehrzahl von Wegen nachgewiesen wurde, die Detektoreinrichtung konfiguriert ist zum Einstellen einer Prioritätsreihenfolge der Wege, basierend auf einem vorbestimmten Kriterium.

6. Bildverarbeitungsvorrichtung nach einem der Ansprüche 1 bis 5, bei der dann, wenn eine Zone außerhalb des vorbestimmten Bewegungsbereichs als Durchstechzone von der zweiten Spezifikationseinrichtung spezifiziert wurde, die Detektoreinrichtung konfiguriert ist zum Detektieren eines Wegs, der nicht durch die Durchstechzone hindurchgeht, an der Zone außerhalb des vorbestimmten Bewegungsbereichs.

7. Radiographiebild-Aufnahmesystem, umfassend:
eine Radiographiebild-Aufnahmeeinrichtung, umfassend:
eine Radiographiebild-Detekteoreinrichtung,
einen Strahlungsabgabeabschnitt, konfiguriert, um der Radiographiebild-Detektoreinrichtung gegenüberzuliegen, und konfiguriert zum Abgeben von Strahlung aus einer Mehrzahl von Positionen während einer Bewegung über eine Mehrzahl von Positionen derart, dass die Strahlung unter unterschiedlichen Winkeln bezüglich des auf der Radiographiebild-Detektoreinrichtung befindlichen Bildobjekts abgegeben wird, und
eine Treibereinrichtung, konfiguriert zum Antreiben einer Biopsienadel zum Entnehmen einer Probe eines interessierenden Objekts innerhalb des Bildobjekts; und
eine Bildverarbeitungsvorrichtung nach einem der Ansprüche 1 bis 6, konfiguriert zum Beseitigen eines Wegs der Biopsienadel basierend auf den mehreren Radiographiebildern, die von der Radiographiebild-Aufnahmeeinrichtung aufgenommen wurden.

8. Bildverarbeitungsverfahren, umfassend:
Erzeugen einer Mehrzahl von Tomographiebildern eines Bildobjekts, bei dem es sich um eine Stelle in einem menschlichen Körper handelt, durch Rekonstruieren einer Mehrzahl von Radiographiebildern, die von einer Radiographiebild-Detektoreinrichtung unter Verwendung einer Detektorfläche der Radiographiebild-Detektoreinrichtung als Referenz erfasst wurden, wobei die mehreren Radiographiebilder erfasst wurden durch Bewegen eines Strahlungsabgabeabschnitts gegenüber der Radiographiebild-Detektoreinrichtung über eine Mehrzahl von Positionen und Bestrahlen des auf der Radiographiebild-Detektoreinrichtung befindlichen Bildobjekts mit Strahlung aus jeder der mehreren Positionen derart, dass die Strahlung unter verschiedenen Winkeln bezüglich des Bildobjekts aufgestrahlt wurde;
Anzeigen auf einer Anzeigeeinrichtung mindestens eines Tomographiebilds der mehreren erzeugten Tomographiebilder, in denen ein interessierendes Objekt innerhalb des Bildobjekts aufgenommen wurde, und Empfangen einer ersten Spezifikation einer Position des interessierenden Objekts in dem mindestens einen Tomographiebild, welches basierend auf der Anzeige spezifiziert wird;
Anzeigen auf der Anzeigeeinrichtung mindestens eines der Tomographiebilder einschließlich einer Zone aus dem interessierenden Objekts auf einer Seite, von der die Biopsienadel einzuführen ist, basierend auf der spezifizierten Position des interessierenden Objekts und einer Einführrichtung der Biopsienadel, und Empfangen einer zweiten Spezifikation einer Soll-Durchstechzone der Biopsienadel, einer durchstichfreien Zone, durch die die Biopsienadel nicht hindurchgehen sollte, oder einer Kombination derselben, spezifiziert auf der Grundlage der Anzeige;
Detektieren eines Wegs der durch die Durchstechzone hindurchgehenden und das interessierende Objekt erreichenden Biopsienadel, wobei die Soll-Durchstechzone als die Durchstechzone betrachtet wird, wenn eine Soll-Durchstechzone spezifiziert wurde, und einer von der durchstichfreien Zone verschiedenen Zone als die Durchstechzone betrachtet wird, wenn eine durchstichfreie Zone spezifiziert wurde; und
Ausgeben einer Warnung dann, wenn der detektierte Weg außerhalb eines vorbestimmten Bewegungsbereichs der Biopsienadel liegt.

9. Bildverarbeitungsverfahren nach Anspruch 8, bei der der vorbestimmte Bewegungsbereich einen Bewegungsbereich enthält, der nach Maßgabe der Möglichkeiten einer Treibereinrichtung zum Antreiben der Biopsienadel vorbestimmt ist, einen Bewegungsbereich, der derart vorbestimmt wurde, dass die Biopsienadel nicht durch eine vorbestimmte Stelle in dem Bildobjekt hindurchgeht, oder eine Kombination derselben.

10. Bildverarbeitungsverfahren nach Anspruch 8 oder 9, bei dem das Anzeigen des mindestens einen Tomographiebilds das Anzeigen des Tomographiebilds und einer Zone umfasst, welche den vorbestimmten Bewegungsbereich auf der Anzeigeeinrichtung repräsentiert.

11. Bildverarbeitungsverfahren nach einem der Ansprüche 8 bis 10, bei dem dann, wenn eine Mehrzahl von interessierenden Objekten bei der ersten Spezifikation spezifiziert wurde, das Detektieren ein Detektieren eines Wegs umfasst, der durch die Durchstechzone hindurchgeht und mindestens zwei interessierende Objekte unter den mehreren interessierenden Objekten erreicht.

12. Bildverarbeitungsverfahren nach einem der Ansprüche 8 bis 11, bei dem dann, wenn mehrere Wege detektiert wurden, das Detektieren das Einstellen einer Prioritätsreihenfolge der Wege basierend auf einem vorbestimmten Kriterium umfasst.

13. Bildverarbeitungsverfahren nach einem der Ansprüche 8 bis 12, bei dem, wenn eine Zone außerhalb des vorbestimmten Bewegungsbereichs als die Durchstechzone bei der zweiten Spezifikation spezifiziert wurde, das Detektieren ein Detektieren eines Wegs umfasst, der nicht durch die Durchstechzone in einer Zone außerhalb des vorbestimmten Bewegungsbereichs hindurchgeht.

14. Nicht-Flüchtiges, computer-lesbares Speichermedium, das ein Programm speichert, welches bei Ausführen durch eine Vorrichtung nach Anspruch 1 diese veranlasst, das Verfahren nach Anspruch 8 auszuführen.

## Revendications

1. Dispositif de traitement d'images, comprenant :
un moyen de génération d'images tomographiques configuré pour générer une pluralité d'images tomographiques d'un sujet d'imagerie, lequel est un site sur un corps humain, en reconstruisant une pluralité d'images radiographiques acquises à partir d'un dispositif de détection d'images radiographiques à l'aide d'une face de détection du dispositif de détection d'images radiographiques comme référence, la pluralité d'images radiographiques étant acquises en déplaçant une section d'irradiation par rayonnement faisant face au dispositif de détection d'images radiographiques à travers une pluralité de positions et en irradiant le sujet d'imagerie disposé sur le dispositif de détection d'images radiographiques avec un rayonnement à partir de chaque position parmi la pluralité de positions, de telle sorte que le rayonnement est irradié suivant différents angles par rapport au sujet d'imagerie ;
un premier moyen de spécification configuré pour afficher, sur un moyen d'affichage, au moins une image tomographique de la pluralité d'images tomographiques, où un objet d'intérêt dans le sujet d'imagerie a été capturé, et lequel permet à un utilisateur de spécifier une position de l'objet d'intérêt dans la au moins une image tomographique sur la base de l'affichage ;
un second moyen de spécification configuré pour afficher, sur le moyen d'affichage, au moins une image parmi la pluralité d'images tomographiques incluant une région allant de l'objet d'intérêt à un côté à partir duquel une aiguille de biopsie doit être introduite, sur la base de la position de l'objet d'intérêt et d'une direction d'introduction de l'aiguille de biopsie, et lequel permet à l'utilisateur de spécifier, sur la base de l'affichage, une région de traversée souhaitée de l'aiguille de biopsie, une région sans traversée à travers laquelle il n'est pas souhaitable que l'aiguille passe, ou une combinaison de celles-ci ;
un moyen de détection configuré pour détecter un trajet de l'aiguille de biopsie traversant la région de traversée et atteignant l'objet d'intérêt, dans lequel la région de traversée souhaitée est considérée comme la région de traversée si une région de traversée souhaitée a été spécifiée, et une région autre que la région sans traversée est considérée comme la région de traversée si une région sans traversée a été spécifiée, et
un moyen d'avertissement configuré pour émettre un avertissement si le trajet détecté est hors d'une plage de déplacement prédéterminée de l'aiguille de biopsie.

2. Dispositif de traitement d'images selon la revendication 1, dans lequel la plage de déplacement prédéterminée inclut une plage de déplacement prédéterminée conformément aux capacités d'un moyen d'entraînement pour entraîner l'aiguille de biopsie, une plage de déplacement prédéterminée telle que l'aiguille de biopsie ne traverse pas un site prédéterminé dans le sujet d'imagerie, ou une combinaison de celles-ci.

3. Dispositif de traitement d'images selon la revendication 1 ou 2, dans lequel le second moyen de configuration est spécifié pour afficher, sur le moyen d'affichage, l'image tomographique et une région représentant la plage de mouvement prédéterminée.

4. Dispositif de traitement d'images selon l'une quelconque des revendications 1 à 3, dans lequel si une pluralité d'objets d'intérêt a été spécifiée par le premier moyen de spécification, le moyen de détection est configuré pour détecter un trajet traversant la région de traversée et atteignant au moins deux objets d'intérêt parmi la pluralité d'objets d'intérêt.

5. Dispositif de traitement d'images selon l'une quelconque des revendications 1 à 4, dans lequel si une pluralité de trajets a été détectée, le moyen de détection est configuré pour régler un ordre de priorité des trajets sur la base d'un critère prédéterminé.

6. Dispositif de traitement d'images selon l'une quelconque des revendications 1 à 5, dans lequel si une région hors de la plage de déplacement prédéterminée a été spécifiée comme région de traversée par le second moyen de spécification, le moyen de détection est configuré pour détecter un trajet ne traversant pas la région de traversée sur la région hors de la plage de déplacement prédéterminée.

7. Système de capture d'images radiographiques, comprenant :
un dispositif de capture d'images radiographiques, comprenant :
un dispositif de détection d'images radiographiques ;
une section d'irradiation par rayonnement configurée pour faire face au dispositif de détection d'images radiographiques, et configurée pour irradier avec un rayonnement à partir de chaque position parmi la pluralité de positions tout en se déplaçant à travers la pluralité de positions, de telle sorte que le rayonnement est irradié suivant différents angles par rapport à un sujet d'imagerie disposé sur le dispositif de détection d'images radiographiques, et
un moyen d'entraînement configuré pour entraîner une aiguille de biopsie pour prélever un échantillon d'un objet d'intérêt dans le sujet d'imagerie, et
le dispositif de traitement d'images selon l'une quelconque des revendications 1 à 6, configuré pour effacer un trajet de l'aiguille de biopsie sur la base de la pluralité d'images radiographiques capturées par le dispositif de capture d'images radiographiques.

8. Procédé de traitement d'images, comprenant les étapes consistant à :
générer une pluralité d'images tomographiques d'un sujet d'imagerie, lequel est un site sur un corps humain, en reconstruisant une pluralité d'images radiographiques acquises à partir d'un dispositif de détection d'images radiographiques à l'aide d'une face de détection du dispositif de détection d'images radiographiques comme référence, la pluralité d'images radiographiques étant acquises en déplaçant une section d'irradiation par rayonnement faisant face au dispositif de détection d'images radiographiques à travers une pluralité de positions, et en irradiant le sujet d'imagerie disposé sur le dispositif de détection d'images radiographiques avec un rayonnement à partir de chaque position parmi la pluralité de positions, de telle sorte que le rayonnement est irradié suivant différents angles par rapport au sujet d'imagerie ;
afficher, sur un moyen d'affichage, au moins une image tomographique de la pluralité d'images tomographiques générées, où un objet d'intérêt dans le sujet d'imagerie a été capturé, et recevoir une première spécification d'une position de l'objet d'intérêt dans la au moins une image tomographique, laquelle est spécifiée sur la base de l'affichage ;
afficher, sur le moyen d'affichage, au moins une image parmi la pluralité d'images tomographiques incluant une région allant de l'objet d'intérêt à un côté à partir duquel une aiguille de biopsie doit être introduite sur la base de la position de l'objet d'intérêt et d'une direction d'introduction d'une aiguille de biopsie, et recevoir une seconde spécification d'une région de traversée souhaitée de l'aiguille de biopsie, d'une région sans traversée à travers laquelle il n'est pas souhaitable que l'aiguille passe, ou d'une combinaison de celles-ci, laquelle est spécifiée sur la base de l'affichage ;
détecter un trajet de l'aiguille de biopsie traversant la région de traversée et atteignant l'objet d'intérêt, dans lequel la région de traversée souhaitée est considérée comme la région de traversée si une région de traversée souhaitée a été spécifiée, et une région autre que la région sans traversée est considérée comme la région de traversée si une région sans traversée a été spécifiée, et
émettre un avertissement lorsque le trajet détecté est hors d'une plage de déplacement prédéterminée de l'aiguille de biopsie.

9. Procédé de traitement d'images selon la revendication 8, dans lequel la plage de déplacement prédéterminée inclut une plage de déplacement prédéterminée conformément aux capacités d'un moyen d'entraînement pour entraîner l'aiguille de biopsie, une plage de déplacement prédéterminée de telle sorte que l'aiguille de biopsie ne traverse pas un site prédéterminé dans le sujet d'imagerie, ou une combinaison de celles-ci.

10. Procédé de traitement d'images selon la revendication 8 ou 9, dans lequel l'étape pour afficher la au moins une image tomographique comprend l'affichage de l'image tomographique et d'une région représentant la plage de déplacement prédéterminée sur le moyen d'affichage.

11. Procédé de traitement d'images selon l'une quelconque des revendications 8 à 10, dans lequel si une pluralité d'objets d'intérêt a été spécifiée dans la première spécification, l'étape pour détecter comprend la détection d'un trajet traversant la région de traversée et atteignant au moins deux objets d'intérêt parmi la pluralité d'objets d'intérêt.

12. Procédé de traitement d'images selon l'une quelconque des revendications 8 à 11, dans lequel si une pluralité de trajets a été détectée, la détection comprend le réglage d'un ordre de priorité des trajets sur la base d'un critère prédéterminé.

13. Procédé de traitement d'images selon l'une quelconque des revendications 8 à 12, dans lequel si une région hors de la plage de déplacement prédéterminée a été spécifiée comme la région de traversée dans la seconde spécification, la détection comprend la détection d'un trajet ne traversant pas la région de traversée sur la région hors de la plage de déplacement prédéterminée.

14. Support de stockage lisible par ordinateur non transitoire, stockant un programme, lequel lorsqu'il est exécuté par un dispositif selon la revendication 1, fait en sorte que ledit dispositif réalise le procédé selon la revendication 8.
